# EUROPEAN PATENT SPECIFICATION

(11) **EP 4 609 793 B1**
(45) Date of publication and mention of the grant of the patent: **16.09.2026**
(21) Application number: 25160866.7
(22) Date of filing: 28.02.2025
(51) Int. Cl.: A61B 6/00

(54) **METHODS AND SYSTEMS FOR COMPUTED TOMOGRAPHY (CT) IMAGING**
VERFAHREN UND SYSTEME ZUR COMPUTERTOMOGRAFIE (CT)-BILDGEBUNG
PROCÉDÉS ET SYSTÈMES D'IMAGERIE PAR TOMOGRAPHIE PAR ORDINATEUR (CT)

(30) Priority: 28.02.2024 CN 202410219680
(43) Date of publication of application: 03.09.2025
(73) Proprietor: Shanghai United Imaging Healthcare Co., Ltd., Shanghai 201807 (CN)
(72) Inventor: MA, Jinglu, Shanghai (CN); WANG, Wenying, Shanghai (CN); LIU, Xiaojuan, Shanghai (CN); FU, Jianwei, Shanghai (CN); ZHANG, Wenda, Shanghai (CN)
(74) Representative: Wang, Bo

(56) References cited:
- US-A1- 2016 287 205
- US-A1- 2018 263 576
- US-A1- 2022 296 202

## Description

### CROSS-REFERENCE TO RELATED APPLICATIONS

This application claims priority to Chinese Application No. 202410219680.X, filed on February 28, 2024.

### TECHNICAL FIELD

The present disclosure relates to the field of medical imaging, and in particular to a method and system for computed tomography (CT) imaging.

### BACKGROUND

Photon-Counting computed tomography (PCCT) has gradually become one of the important development directions of CT technology with its higher spatial resolution, lower electronic noise, lower radiation dose, and more accurate spectral decomposition capability.

US 2022/296202 A1 describes a calibration method for photon-counting CT systems where a forward model's response functions and pileup terms are refined by comparing measured photon counts against expected ground-truth values from known material slabs prior to basis material decomposition.

The embodiments of the present disclosure provide a method and system for CT imaging to obtain medical images of higher quality.

### SUMMARY

The invention is defined in independent claims 1, 10 and 13. Particular embodiments are defined in the dependent claims.

### BRIEF DESCRIPTION OF THE DRAWINGS

The present disclosure will be further illustrated by way of exemplary embodiments, which will be described in detail by means of the accompanying drawings. These embodiments are not limiting, and in these embodiments, the same numbering indicates the same structure, wherein:
FIG. 1 is a schematic diagram illustrating an application scenario of a system for CT imaging according to some embodiments of the present disclosure;
FIG. 2 is a flowchart illustrating an exemplary method for CT imaging according to some embodiments of the present disclosure;
FIG. 3 is a flowchart illustrating an exemplary process of determining an optimization parameter according to some embodiments of the present disclosure;
FIG. 4 is a flowchart illustrating an exemplary process of determining an optimization parameter according to some embodiments of the present disclosure;
FIG. 5 is a flowchart illustrating an exemplary process of generating a first target image according to some embodiments of the present disclosure;
FIG. 6 is a flowchart illustrating an exemplary process of generating a second target image according to some embodiments of the present disclosure;
FIG. 7 is a flowchart illustrating an exemplary process of generating a target image according to some embodiments of the present disclosure;
FIG. 8 is a flowchart illustrating an exemplary method for CT imaging according to some embodiments of the present disclosure; and
FIG. 9 is a block diagram illustrating an exemplary system for CT imaging according to some embodiments of the present disclosure.

### DETAILED DESCRIPTION

In order to more clearly illustrate the technical solutions of the embodiments of the present disclosure, the accompanying drawings required to be used in the description of the embodiments are briefly described below. Obviously, the accompanying drawings in the following description are only some examples or embodiments of the present disclosure, and it is possible for a person of ordinary skill in the art to apply the present disclosure to other similar scenarios in accordance with these drawings without creative labor. Unless obviously obtained from the context or the context illustrates otherwise, the same numeral in the drawings refers to the same structure or operation.

It should be understood that the terms "system", "device", "unit" and/or "module" used herein are a way to distinguish between different components, elements, parts, sections, or assemblies at different levels. However, the terms may be replaced by other expressions if other words accomplish the same purpose.

As shown in the present disclosure and in the claims, unless the context clearly suggests an exception, the words "one," "a," "an," "one kind," and/or "the" do not refer specifically to the singular, but may also include the plural. Generally, the terms "including" and "comprising" suggest only the inclusion of clearly identified steps and elements, however, the steps and elements that do not constitute an exclusive list, and the method or apparatus may also include other steps or elements.

Flowcharts are used in the present disclosure to illustrate the operations performed by a system according to embodiments of the present disclosure, and the related descriptions are provided to aid in a better understanding of the magnetic resonance imaging method and/or system. It should be appreciated that the preceding or following operations are not necessarily performed in an exact sequence. Instead, steps can be processed in reverse order or simultaneously. Also, it is possible to add other operations to these processes or to remove a step or steps from these processes.

Compared with an energy integrating detector of traditional CT, a Photon-Counting detector uses a new semiconductor detector that can effectively detect energy of each incident photon and record the energy in pre-divided energy intervals, and can measure attenuation in different energy intervals, thereby achieving excellent spectral analysis capacity and perfect data matching effect.

In some embodiments of the present disclosure, material decomposition is performed on scanning data in a projection domain (also referred to as non-image domain). Compared with material decomposition in an image domain of PCCT, material decomposition in the projection domain can achieve a better hardening artifact removal effect by effectively using the above advantages of the Photon-Counting detector, thereby achieving more accurate material quantitative analysis, and obtaining better image quality.

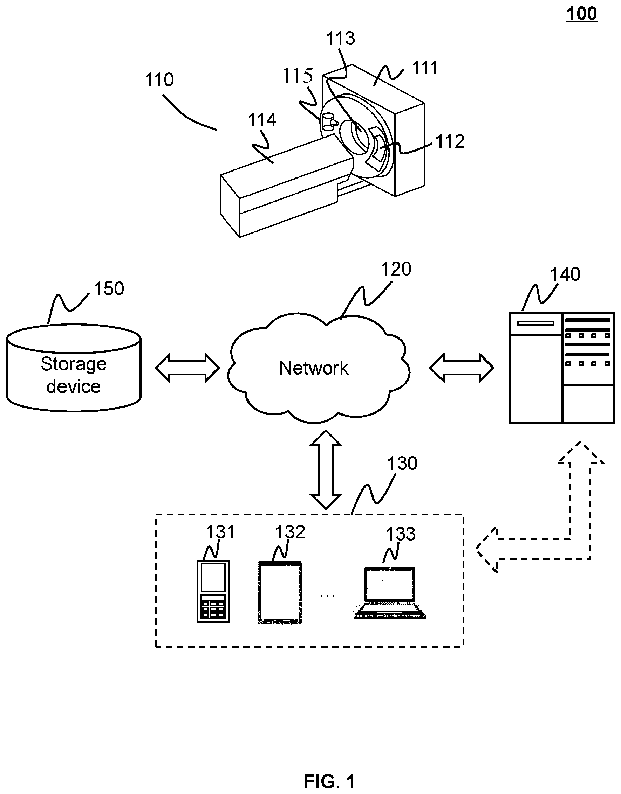

FIG. 1 is a schematic diagram illustrating an application scenario of a system for CT imaging according to some embodiments of the present disclosure.

As shown in FIG. 1, a system for CT imaging 100 may include an imaging device 110, a network 120, a terminal 130, a processing device 140, and a storage device 150.

The imaging device 110 may be configured to obtain scanning data/images by scanning a target subject. In some embodiments, the imaging device 110 may be a medical imaging device, the imaging device 110 may be a single-modal imaging device, such as a computed tomography (CT) imaging device, the imaging device 110 may be a multi-modal imaging device, such as a single photon emission computed tomography- computed tomography (SPECT-CT) imaging device, a positron emission tomography- computed tomography (PET-CT) imaging device, a magnetic resonance computed tomography-computed tomography (MR-CT) imaging device, etc. In some embodiments, the imaging device 110 may include a gantry 111, a detector 112, a scanning region 113, a scanning bed 114, and a radiation source 115. A target subject may be placed on the scanning bed 114 and moved to the scanning region 113 to be scanned. The gantry 111 may support the radiation source 115 and the detector 112. The radiation source 115 may be configured to emit radiation. The radiation used herein may include a particle ray, a photon ray, etc. The particle ray may include neutron, proton, electron, µ-meson, heavy ion, α-ray, or the like, or any combination thereof. The photon ray may include X-ray, γ-ray, ultraviolet, laser, or the like, or any combination thereof. For brevity, X-ray may be taken as an example in the description below. In some embodiments, the detector 112 may include one or more detector units arranged in an array. The one or more detector units may be and/or include a single-row detector and/or a multi-row detector. The one or more detector units may include a semiconductor detector and other detectors (e.g., a scintillation detector), etc. The detector 112 may be a Photon-Counting detector. After the X-rays reach the detector 112, the detector 112 may directly measure a count of photons, a Photon-Counting rate, an energy of each photon, an arrival time of each photon, etc.

The network 120 may include any suitable network that facilitates information and/or data exchange of the imaging device 110. In some embodiments, at least one component (e.g., the imaging device 110, the terminal 130, the processing device 140, and the storage device 150) of the system for CT imaging 100 may exchange information and/or data with at least one other component of the system for CT imaging 100 via the network 120. For example, the processing device 140 may obtain the scanning data of the target subject from the imaging device 110 via the network 120. In some embodiments, the network 120 may include at least one network access point. For example, the network 120 may include a wired and/or wireless network access point (e.g., a base station and/or an Internet exchange point), and the at least one component of the system for CT imaging 100 may be connected to the network 120 through the access point to exchange data and/or information.

The terminal 130 may be configured to communicate with and/or be connected with the imaging device 110, the processing device 140 and/or the storage device 150. In some embodiments, the terminal 130 may include a mobile device 131, a tablet computer 132, a laptop computer 133, or the like, or any combination thereof. For example, the mobile device 131 may include a mobile control handle, a personal digital assistant (PDA), a smart phone, or the like, or any combination thereof. In some embodiments, the terminal 130 may include a display device, such as a display. The display device may be configured to display a finally obtained target image, such as a medical image of a patient, a three-dimensional model, or an operation panel related to medical imaging, etc. In some embodiments, the terminal 130 may be part of the processing device 140.

The processing device 140 may be configured to process data and/or information obtained from the imaging device 110, the terminal 130, the storage device 150, or other components of the system for CT imaging 100. For example, the processing device may be configured to perform one or more operations in the method for CT imaging disclosed in some embodiments of the present disclosure. In some embodiments, the processing device 140 may include a single server or a server group. The server group may be centralized or distributed. In some embodiments, the processing device 140 may be local or remote. For example, the processing device 140 may access information and/or data from the imaging device 110, the storage device 150, and/or the terminal 130 via the network 120. As another example, the processing device 140 may be directly connected with the imaging device 110, the terminal 130, and/or the storage device 150 to access information and/or data. As another example, the processing device 140 may be disposed on the imaging device 110. In some embodiments, the processing device 140 may be implemented on a cloud platform. For example, the cloud platform may include a private cloud, a public cloud, a hybrid cloud, a community cloud, a distributed cloud, a cloud-to-cloud, a multi-cloud, or the like, or any combination thereof.

The storage device 150 may be configured to store data, instructions and/or any other information. For example, the storage device 150 may store data obtained from the imaging device 110, the terminal 130 and/or the processing device 140. In some embodiments, the storage device 150 may store data and/or instructions used by the processing device 140 to execute or use to complete the exemplary methods described in the present disclosure. In some embodiments, the storage device 150 may include a mass memory, a removable memory, a volatile read-write memory, a read-only memory (ROM), or the like, or any combination thereof. In some embodiments, the storage device 150 may be implemented on a cloud platform.

In some embodiments, the storage device 150 may be connected to the network 120 to communicate with the at least one other component (e.g., the processing device 40, and the terminal 130) of the system for CT imaging 100. The at least one component of the system for CT imaging 100 may access data stored in the storage device 150 via the network 120. In some embodiments, the storage device 150 may be part of the processing device 140. In some embodiments, the processing device 140 and the storage device 150 may be integrated in the imaging device 110.

It should be noted that the above description is provided for illustrative purposes only and is not intended to limit the scope of the present disclosure. For those having ordinary skills in the art, various changes and modifications may be made under the guidance of the contents of the present disclosure. The features, structures, methods and other features of the exemplary embodiments described in the present disclosure may be combined in various ways to obtain additional and/or alternative exemplary embodiments. For example, the storage device 150 is a data storage device including a cloud computing platform, such as a public cloud, a private cloud, a community, and a hybrid cloud. However, these changes and modifications will not deviate from the scope of the present disclosure.

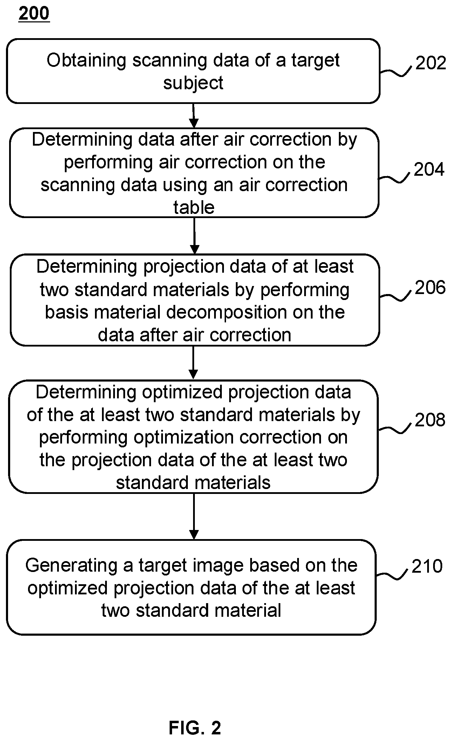

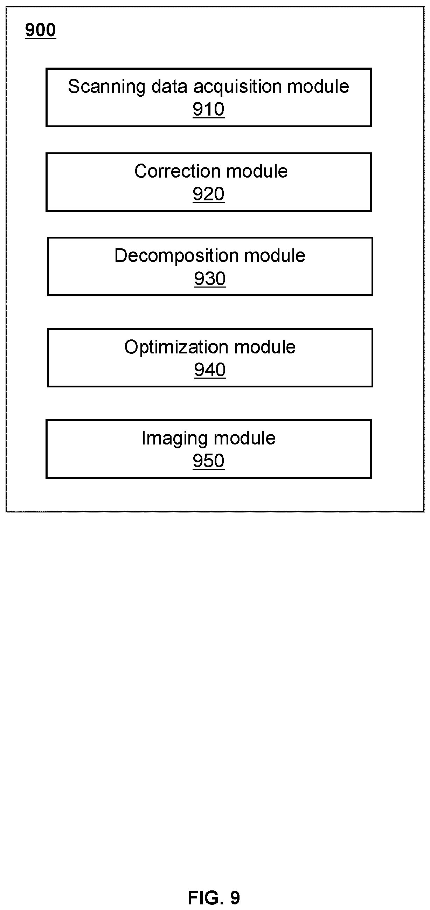

FIG. 2 is a flowchart illustrating an exemplary method for CT imaging according to some embodiments of the present disclosure. In some embodiments, a process 200 may be performed by a system for CT imaging (e.g., a system for CT imaging 900 in FIG. 9) or a processing device (e.g., the processing device 140). As shown in FIG. 2, the process 200 may include the following operations.

In 202, scanning data of a target subject may be obtained.

The target subject may include the whole or part of a biological subject and/or non-biological subject involved in a scanning process. For example, the target subject (also referred to as a scanning subject) is a living or non-living organic and/or inorganic substance, such as the head, ear, nose, mouth, neck, chest, abdomen, liver, gallbladder, pancreas, spleen, kidney, spine, etc.

The scanning data refers to data that can be used for imaging and is obtained by scanning a target subject using an imaging device. For example, the scanning data may be projection data obtained by the detector.

In some embodiments, the scanning data may be obtained by scanning the target subject using an imaging device (e.g., a CT device). In some embodiments, the scanning data may be pre-scanned and stored in a database, a storage device, or the imaging device, and the scanning data may be obtained by reading the scanning data.

In some embodiments, the scanning data of the target subject may include data obtained by scanning using a PCCT device, or data obtained using a dual-energy CT (DECT), such as data obtained by dual-source CT (DSCT), a dual-layer spectral detector, an fast kVp switching technology, or sequential kVp switching acquisition on a single-energy CT (SECT) system, which is not limited in this embodiment. For example, the radiation source and the detector may rotate to scan the target subject from at least one projection angle. For each of the at least one projection angle, the detector may output a set of projection data. The scanning data may include the projection data corresponding to the at least one projection angle. The scanning data may include at least one of a count of photons, a Photon-Counting rate, energy of each photon, and arrival time of each photon of the X-rays recorded by the Photon-Counting detector.

A set of projection data corresponding to each projection angle may be represented in a matrix format. For example, a set of projection data may be represented in a 912*64 matrix, and each element in the matrix corresponds to a pixel (e.g., a detection unit) of the detector. Each of positions of the elements in the matrix may correspond to one of positions of the pixels of the detector. Each element in the matrix may include the count of photons, the Photon-Counting rate, the energy of each photon, and the arrival time of each photon detected by the corresponding pixel in the detector. The size of the matrix depends on a count of channels and rows of a detector array of the imaging device. For example, 912 may be the count of channels of the detector array, and 64 may be the count of rows of the detector array.

In 204, data after air correction may be determined by performing air correction on the scanning data using an air correction table.

In some embodiments, performing air correction on the scanning data using an air correction table may be considered as data preprocessing on the scanning data.

The data preprocessing refers to various operations performed before performing a specific task on the scanning data. In some embodiments, the data preprocessing may include performing data cleaning, data correction, and data conversion on the scanning data. The specific task may include performing basis material decomposition on the scanning data, image reconstruction based on the scanning data, etc.

Taking data correction as an example, the data preprocessing may include performing air correction on the scanning data using the air correction table. The air correction table refers to reference data obtained by scanning the air. During CT scanning, a Photon-Counting detector is affected by scattering and absorption from rays, which may cause artifacts or deviations in a final CT image. Air correction eliminates possible artifacts through additional air scans or specific algorithms. For example, an air scan is performed when there is no scanning subject in the field of view (FOV) of the imaging device to obtain reference data by scanning the air.

In some embodiments, the air calibration table may be generated based on daily collected air data and air data collected during system calibration. Specifically, the air correction table represents a ratio of the daily collected air data to the air data collected during the system calibration. More descriptions regarding the system calibration, daily collected air data, and air data collected during the system calibration may be found in the present disclosure below.

In some embodiments, the air correction table may be stored in a table or other form, such as a curve, etc., which is not limited in the resent disclosure. For example, the air correction table may be represented by the following Formula (1): $gain _ map = {Air}_{Corr} / {Air}_{cali}$ where *gain_map* denotes the air correction table, *Air_{Corr}* denotes the daily collected air data, and *Air_{cali}* denotes the air data collected during the system calibration.

In some embodiments, the processing device may calculate and record the ratio of the daily collected air data to the air data collected during the system calibration to obtain the air correction table. For example, the air correction table may be obtained by periodically collecting air data and calculating the ratio.

For example, the air correction table may be obtained by: obtaining current air data corresponding to the scanning data; obtaining reference air data corresponding to system calibration; and determining the air correction table based on the current air data and the reference air data.

When there is no scanning subject in FOV of the imaging device, the radiation source may emit X-rays at a certain projection angle, and the detector may receive the X-rays to obtain the projection data. This scanning process is referred to as air scanning, and the obtained projection data is referred to as air data. Daily air scanning may be performed between the latest system calibration and obtaining the scanning data. The current air data corresponding to the scanning data refers to the air data obtained from the closest daily air scanning to the acquisition of the scanning data. In some embodiments, the air scanning may be performed (e.g., 5 min, 10 min, 30 min, 1 h, etc.) before obtaining the scanning data, and the obtained air data may be the current air data corresponding to the scanning data. For example, a spatial environment condition of the air scanning performed when the current air data is obtained is the same as or close to a spatial environment condition when the scanning data is obtained. The spatial environment condition may include a temperature, a humidity, an air composition ratio in the space where the imaging device is located. The spatial environment condition being close to the spatial environment condition when the scanning data is obtained means that the change in the spatial environment condition does not exceed a preset change threshold. For example, the change in the spatial environment condition does not exceed 3%, 5%, 8%, etc.

The reference air data corresponding to the system calibration refers to the air data collected by performing air scanning when the system calibration is performed. In some embodiments, the reference air data may be obtained and stored when the system calibration is performed, and the processing device may directly obtain the reference air data by querying from the stored data related to the system calibration. The projection angle corresponding to the reference air data and the current air data may be the same or different.

In some embodiments, an X-ray coverage and/or a distance between the radiation source and the detector may be consistent in the air scanning performed during the system calibration and the daily air scanning, such that the consistency of the reference air data with the current air data can be ensured and the accuracy of the air correction table can be improved.

In some embodiments, the specific process of determining the air correction table based on the current air data and the reference air data may include calculating a ratio of the current air data to the reference air data as described above. For example, similar to the scanning data, both the current air data and the reference air data may be represented by a matrix format. For example, the air data may be represented by a matrix of size 912*64, and each element in the matrix represents a pixel (e.g., a detection unit). Each of the positions of the elements in the matrix may correspond to one of the positions of the pixels of the detector. Each element in the matrix may include the count of photon, the Photon-Counting rate, the arrival time of each photon, the energy of each photon, etc. detected by the corresponding pixel of the detector. The size of the matrix depends on a count of channels and rows of a detector array of the imaging device. For example, 912 may be a count of channels of the detector array, and 64 may be a count of rows of the detector array.

Accordingly, the ratio of the current air data to the reference air data may be represented by a ratio of corresponding elements. For example, a ratio of an element of the current air data to the corresponding element of the reference air data may be determined. The data format of the finally determined air correction table may be the same as the data format of the air data, such as data of the size of 912*64. Correspondingly, the obtained scanning data may also be data of the size of 912*64.

In some embodiments, the processing device may perform correction by multiplying the scanning data by the air correction table. For example, the data after air correction is obtained by multiplying the projection data corresponding to each projection angle in the scanning data by the air correction table. As another example, for each element in the projection data corresponding to each projection angle in the scanning data, the processing device may multiply the element of the scanning data by the corresponding element of the air correction table to obtain the data after air correction.

In some embodiments, the data after air correction is also referred to as pre-processed scanning data, or corrected scanning data.

In 206, projection data of at least two standard materials may be determined by performing basis material decomposition on the data after air correction.

In some embodiments, the processing device may determine the projection data of the at least two standard materials by performing basis material decomposition on the data after air correction based on a response model parameter.

The response model parameter is a parameter used to describe features and behaviors of the Photon-Counting detector. In a Photon-Counting CT system, individual detector units or detection channels in a detector array measure an intensity of radiation passing through a patient's body. However, due to some factors (e.g., scattering, absorption, noise, etc.), a measured signal may not completely match an actual incident radiation intensity. A more accurate reconstructed image may be achieved by correcting these effects using the response model parameter.

In some embodiments, the response model parameter may be configured to calibrate a correspondence between a Photon-Counting rate and a path integral length of a material to be decomposed (e.g., a system calibration material). The Photon-Counting rate refers to a count of photons that interact with the detector per unit time (e.g., 1 s, 1 min, 1 h, etc.). The path integral length of the material to be decomposed refers to a path length that X-rays pass through the material to be decomposed, which reflects a penetration depth of the X-rays inside the target subject and may be used to estimate a density or tissue structure inside the target subject. Under a fixed bulb tube emission light intensity, the correspondence reflects the correlation between the Photon-Counting rate and the path integral length of the material to be decomposed. For example, at a certain tube current (mA), a certain Photon-Counting rate corresponds to a certain path integral length of a material to be decomposed.

In some embodiments, the processing device may obtain the response model parameter by using a projection domain calibration algorithm (e.g., the system calibration) based on a mathematical model, and the Photon-Counting rate in each energy interval may be expressed as a function of the path integral length of the material to be decomposed. For example, the system calibration may include the following operations.
1. Reference air data may be obtained by performing air scanning. When there is no scanning subject in the FOV of the imaging device, the radiation source may emit X-rays at a certain projection angle, and the detector may receive the X-rays to obtain the reference air data. The reference air data may be found in the related descriptions above.
2. At least two system calibration materials with known density or tissue composition may be obtained, e.g., polymethyl methacrylate (PMMA), aluminum, water, fat, calcium salts, etc. Merely by way of example, PMMA and aluminum in plate shapes of different thicknesses may be obtained. At least two system calibrating materials with different thicknesses may be combined (e.g., the at least two system calibrating materials may be stacked in a thickness direction) to obtain different thickness combinations. For example, PMMA in a plate shape of two thicknesses (e.g., D1 and D2) and aluminum in a plate shape of two thicknesses (e.g., D3 and D4) may be obtained. The PMMA and the aluminum of different thicknesses may be combined (e.g., the PMMA and the aluminum of different thicknesses may be stacked in the thickness direction) to obtain thickness combination 1 (e.g., D1 and D3), thickness combination 2 (e.g., D1 and D4), thickness combination 3 (e.g., D2 and D3), and thickness combination 4 (e.g., D2 and D4).
3. The system calibration data may be obtained. For each thickness combination, the system calibration material of the thickness combination may be scanned under different scanning conditions using a Photon-Counting CT device. The scanning conditions may include at least one of a ray energy or intensity (e.g., a tube voltage and/or a tube current), a coverage (FOV) of the X-rays emitted by the radiation source, and the distance between the radiation source and the detector. For each scanning condition and each thickness combination, the Photon-Counting rate detected by each detection unit and the corresponding path integral length may be recorded. The path integral length corresponding to the Photon-Counting rate detected by one detection unit refers to a path length of the X-rays received by the detection unit passing through at least two system calibration materials, which may be obtained according to the thicknesses of the at least two system calibration materials, and a relative position relationship among the radiation source, the detector, and the at least two calibration materials. The system calibration data may include the Photon-Counting rate recorded during the system calibration and the corresponding path integral length (also referred to as the actual path integral length). The system calibration data may include the reference air data, the Photon-Counting rate recorded by scanning different thickness combinations and the corresponding path integral length. The reference air data may also include the Photon-Counting rate corresponding to each detection unit and the path integral length (e.g., 0).
4. A response model may be established. A mathematical model representing the relationship between the Photon-Counting rate and the path integral length of the material to be decomposed may be established through statistical analysis and mathematical modeling based on the system calibration data. The data model may be a simple linear function, a polynomial function, or a more complex model. Parameters of the mathematical model may include the response model parameter.

In some embodiments, for each thickness combination, the processing device may scan the thickness combination in an ascending order of mA (i.e., the tube current) under each system parameter according to different kV (i.e., the tube voltage), bowtie (i.e., a filter for adjusting the intensity of the X-rays), and other system parameters to obtain a plurality of combinations of Photon-Counting rate-path integral length. Finally, the parameters of the response model may be determined by fitting the reference air data and the plurality of combinations of Photon-Counting rate-path integral length.

In some embodiments, each detector unit may correspond to a response model parameter. Taking a detection unit as an example, the established response model may be expressed by the following Formula (2). ${y}_{b} = f \left({L}_{1}, {L}_{2}\right)$ where y*_{b}* denotes the Photon-Counting rate in each energy interval detected by the detection unit, *L*₁ and *L*₂ denote a path length of the X-rays detected by the detection unit penetrating through two system calibration materials, respectively, i.e., the path integral length, and *f*(*) denotes the response model parameter corresponding to the detection unit. The response model parameter may be determined by fitting the system calibration data corresponding to the detection unit.

In particular, the response model parameter may be expanded, and may be shown as the following Formula (3) after expansion: ${y}_{b} = \frac{exp \left(-ατ\right)}{1 + ατ} I {\sum }_{p = 0 , 1 , 2} {\left(ατ\right)}^{p} \int dE {S}_{p , b} \left(E\right) exp \left(-{μ}_{1}\left(E\right){L}_{1}-{μ}_{2}\left(E\right){L}_{2}\right)$ where *τ* denotes an equivalent dead time of the detector, *I* denotes a flux value related to mA, *p* denotes a data pile-up order, *µ*₁ and *µ*₂ denote the linear attenuation coefficients of the two system calibration materials, respectively, and *S_{p,b}* denotes a fitting spectrum of a high-order pile-up effect in the current energy interval. S_{ref} denotes a spectral response without pile-up at a low flux, and an incident count rate α without pile-up may be represented by the following Formula (4): $α = \frac{I}{{I}_{ref}} \int dE {S}_{ref , allbin} \left(E\right) exp \left(-{μ}_{1}\left(E\right){L}_{1}-{μ}_{2}\left(E\right){L}_{2}\right)$ where *I_{ref}* denotes a reference flux value.

It should be noted that the above examples are for illustrative purposes only and are not intended to limit the specific form of the response model parameter. The response model parameter may also be expressed in other ways. For example, the response model may be a machine learning model. Each detection unit may correspond to a response model. For one detection unit, the Photon-Counting rate detected by the detection unit in the system calibration data is used as an input in the model training process, and the path integral length of the at least two system calibration materials corresponding to the Photon-Counting rate is used as a label in the model training process to train an initial model to obtain the response model corresponding to the detection unit, and a parameter of the response model is the response model parameter corresponding to the detection unit.

The basis material decomposition refers to a process of expressing attenuation information of a target subject to X-rays by a combination of two or more basis materials (e.g., the system calibration materials) with known attenuation characteristics.

In some embodiments, the projection data of the at least two standard materials refers to a combination of basis materials with known attenuation characteristics obtained by performing the basis material decomposition on the pre-processed scanning data. For example, the projection data of the at least two standard materials may be represented as path integral lengths L of the at least two basis materials.

When the scanning data is obtained by scanning the target subject, for one detection unit, a path of the received X-rays passing through the target subject may be denoted as L. Basis material decomposition refers to a process of expressing the path L by a combination of path integral lengths of two or more basis materials (e.g., the system calibration materials) with known attenuation characteristics. The projection data of the at least two standard materials may include a combination of path integral lengths of two or more system calibration materials corresponding to each detection unit.

In some embodiments, the processing device may perform the basis material decomposition by using the response model parameters corresponding to the detection units of the detector. For example, during the system calibration, polymethyl methacrylate (PMMA) and aluminum may be used as the system calibration materials. The projection data of the at least two standard materials obtained by basis material decomposition of the data after air correction using the response model parameter may be expressed as the path integral lengths of PMMA and aluminum. For example, the processing device may obtain the projection data of the at least two standard materials by performing decomposition calculation using maximum likelihood, least squares, or the maximum likelihood or the least squares with regularization terms.

For example, the maximum likelihood used for basis material decomposition may be shown in the following Formula (5): $ML = {\sum }_{b = 1 , … , N} \left({y}_{b}-{m}_{b}{y}_{b}\right)$ where y*_{b}* denotes a Photon-Counting expected value of each energy interval corresponding to one detection unit, which can be understood as an output value of the response model corresponding to the detection unit by inputting a set of candidate path integral lengths (L_{1c}, L_{2c}) of PMMA and aluminum into the corresponding response model (e.g., Formula (2)); *m_{b}* denotes a measured value of the Photon-Counting rate of the energy interval detected by the detection unit in the scanning data, N denotes the count of the detection units of the detector, and ML denotes a likelihood function value. Formula (5) can be understood as trying different sets of candidate path integral lengths for each detection unit to find a set of candidate path integral lengths such that *y_{b}* determined based on the candidate path integral lengths is closed to *m_{b}*, such as finding a set of candidate path integral lengths that maximizes the likelihood function value. In this case, these selected candidate path integral lengths corresponding to the detection units may be determined as the projection data of the at least two standard materials.

In some embodiments, if the response model is a trained machine learning model corresponding to a detection unit, the measured value of the Photon-Counting rate detected by the detection unit in the scanning data may be input into the response model, and the response model may output the path integral lengths, i.e., the projection data of the at least two standard materials, corresponding to the detection unit.

In some embodiments, the target image may be generated based on the obtained projection data of the at least two standard materials. In some embodiments, the processing device may optimize the projection data of the at least two standard materials, and generate, based on the optimized projection data of the at least two standard materials, the target image. That is, the operation 208 is optional and may be omitted in some embodiments.

In 208, optimized projection data of the at least two standard materials may be determined by performing optimization correction on the projection data of the at least two standard materials.

In some embodiments, performing optimization correction on the projection data of the at least two standard materials is also referred to as performing data post-processing on the projection data of the at least two standard materials. Accordingly, the optimized projection data of the at least two standard materials is referred to as post-processed projection data of the at least two standard materials, which means the projection data of the at least two standard materials obtained after post-processing. Data post-processing refers to a process of optimizing and correcting the data obtained after performing a specific task.

The optimized projection data of the at least two standard materials, i.e., the post-processed projection data of the at least two standard materials, refers to a combination of basis materials obtained after performing optimization correction on the projection data of the at least two standard materials.

The response model may be obtained during the system calibration. In an ideal condition, if the Photon-Counting rate in the system calibration data is input into the determined response model, an output value may be consistent with the path integral length corresponding to the Photon-Counting rate in the system calibration data. However, during the system calibration, when the response model parameter is obtained based on the system calibration data, it is to ensure that the determined response model is close to the overall trend of the system calibration data. So if the Photon-Counting rate in the system calibration data is input into the determined response model, the output value may have a deviation from the path integral length corresponding to the Photon-Counting rate in the system calibration data. The deviation is be considered as a systematic error. The optimization correction is to correct the deviation.

Optimization correction refers to adjusting and optimizing the projection data of the at least two standard materials. For example, assuming that the path integral lengths of two basis materials (*L*₁, *L*₂) in the projection data of the at least two standard materials are 10 and 20, respectively, the path integral lengths of one or two basis materials may be adjusted and optimized through optimization correction. As another example, optimization correction is also referred to as data noise reduction of the projection data of the at least two standard materials in a projection domain.

In some embodiments, the determining optimized projection data of the at least two standard materials by performing optimization correction on the projection data of the at least two standard materials may include: obtaining an optimization parameter that is determined based on system calibration data; and determining the optimized projection data of the at least two standard materials by performing, based on the optimization parameter, the optimization correction on the projection data of the at least two standard materials.

The system calibration material refers to a reference material selected when the system calibration is performed. In some embodiments, by performing the system calibration, a system calibration result may be obtained. The system calibration result may include information such as the system calibration data, the system calibration material, the response model parameter, the optimization parameter, etc.

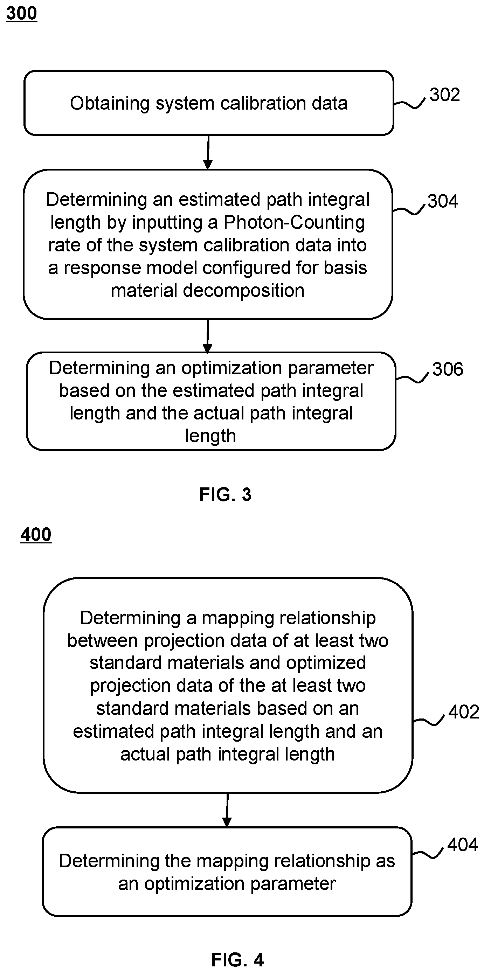

More descriptions regarding the optimization correction may be found in the related descriptions of FIG. 3 and FIG. 4.

The operations 206 and 208 are described by taking projection data corresponding to one projection angle of the scanning data as an example. If the scanning data includes projection data corresponding to two or more projection angles, the operation 206 (i.e., basis material decomposition) and the operation 208 (i.e., optimization correction) may be performed on the projection data corresponding to each projection angle.

In addition, according to the above description of the system calibration, the air correction, the basis material decomposition, and the optimization correction, it can be seen that the system calibration, the air correction, the basis material decomposition, and the optimization correction involved in the method for CT imaging disclosed in the present disclosure are all performed in a projection domain (e.g., projection data obtained by the detector, such as the Photon-Counting rate, etc.), and these operations do not involve image reconstruction, or data in an image domain. Performing operations such as the air correction and the basis material decomposition in the projection domain can effectively reduce the degradation of image quality caused by errors or noise in the reconstruction process. Meanwhile, by performing air correction and optimization correction directly on projection data in projection domain to correct the system error (e.g., system response, air scattering, etc.), error accumulation caused by performing correction in the image domain can be avoided.

In 210, a target image may be generated based on the optimized projection data of the at least two standard materials.

The target image refers to a medical image that is required after a series of procedures. For example, the target image may be a high-energy CT (HECT) image, a low-energy CT (LECT) image, or a virtual monoenergetic image (VMI).

In some embodiments, the processing device may directly generate the target image based on the optimized projection data of the at least two standard materials through image reconstruction. An image reconstruction algorithm may include iterative reconstruction, back-projection reconstruction, neural network reconstruction, etc., which is not limited in this embodiment.

In some embodiments, the processing device may determine a data synthesis result by performing data synthesis on the optimized projection data of the at least two standard materials, and generate the target image based on the data synthesis result.

For example, the processing device may determine the data synthesis result by synthesizing the optimized projection data of the at least two standard materials in a projection domain; and generate the target image based on the data synthesis result.

The data synthesis result refers to data obtained after synthesizing the optimized basic material data. The data synthesis result may include synthesizing other basis material results except decomposition.

Generating the target image based on the data synthesis result may use processes such as iterative reconstruction and back-projection reconstruction, which is not limited in this embodiment.

In some embodiments, the target image generated based on the data synthesis result may include a high-energy image, a low-energy image, a full-energy image, the VMI, etc.

In this embodiment, by synthesizing the projection data of the at least two standard materials in a data domain (e.g., the projection domain), imaging of other spectral images can be achieved while guaranteeing the imaging effect of the images.

It should be noted that, in some embodiments, the processing device may directly perform data synthesis based on the projection data of the at least two standard materials and reconstruct the target image. For example, in a scenario where an image output speed needs to be accelerated and an image quality requirement is relatively low, data post-processing on the projection data of the at least two standard materials may not be required.

In some embodiments, in the process 200, the air correction or the optimization correction may be omitted.

In some embodiments, in order to better apply the method for CT imaging disclosed in the embodiments of the present disclosure to clinical application, the system can be calibrated in a preparation stage before scanning a patient (e.g., before obtaining the scanning data). Specifically, a test scan may be performed on a phantom, or the like, through the operations 202-210 described above, and a test target image may be generated based on a test scan result. Then whether a data preprocessing and/or data post-processing mode needs to be optimized may be determined based on the test target image, and the target image may be obtained based on the optimized data preprocessing and/or the optimized data post-processing, such that the target image with a higher quality can be obtained during clinical application.

In some embodiments, optimizing the data preprocessing mode may include correcting the air correction table, and optimizing the basis material decomposition may include correcting the system calibration result by re-performing system calibration.

The correction refers to correcting the air correction table and/or the system calibration result. For example, for the air correction table, the air data may be collected by re-performing daily air scanning, and the air correction table may be corrected using the newly collected air data. As another example, a corrected system calibration result (e.g., the system calibration data, the system calibration material, the response model parameter, the optimization parameter, etc.) may be obtained by re-performing the system calibration.

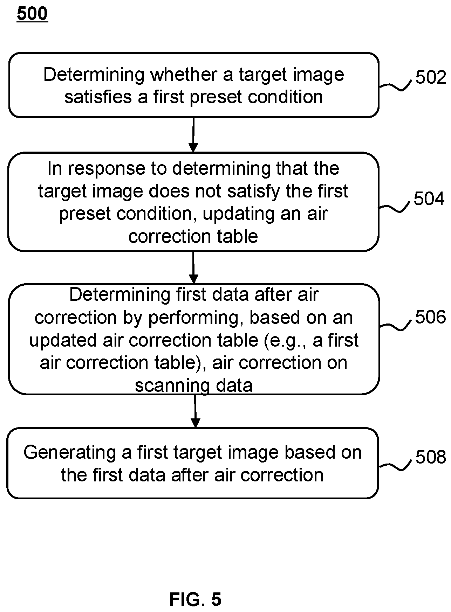

In some embodiments, whether a test target image satisfies a first preset condition (see descriptions in connection with FIG. 5) may be determined. In response to determining that the test target image satisfies the first preset condition, it is determined that there is no need to optimize the data preprocessing and/or data postprocessing process. In response to determining the test target image does not satisfy the first preset condition, it is determined that there is a need to optimize the data preprocessing and/or data postprocessing process. Whether the test target image satisfies the first preset condition may may be determined in various ways, such as manually determining whether the image quality satisfies the requirements, determining whether the image quality satisfies the requirements using a machine learning model, etc., which is not limited in this embodiment.

In some embodiments, if it is determined that the correction is needed, the daily air scanning may be re-performed, and the air correction table may be corrected using newly collected air data. Then image reconstruction may be performed on the scanning data by performing the process 200 based on the corrected air correction table. The operations 202-210 may be performed again based on the corrected air correction table to obtain a new test target image (which is referred to as a first test target image). Then the quality of the first test target image may be determined. If the first test target image satisfies a requirement (e.g., the first preset condition), image reconstruction may be performed on the scanning data by performing the process 200 based on the corrected air correction table. If the first test target image does not satisfy the requirement (e.g., the first preset condition), the system calibration may be re-performed, and image reconstruction may be performed on the scanning data by performing the process 200 based on the corrected air correction table and the corrected system calibration result. In particular, according to the process for determining an air correction table illustrated in operation 204, the air correction table is determined based on daily collected air data and air data collected during system calibration. After daily air scanning is re-performed, new daily collected air data is acquired. A first air correction table is generated based on the new daily collected air data and air data collected during system calibration. Then, after re-performing the system calibration, new air data collected during system calibration and a corrected system calibration result are acquired. A second air correction table is generated based on the new daily collected air data and the new air data collected during system calibration. Image reconstruction may be performed on the scanning data by performing the process 200 based on the second air correction table and the corrected system calibration result.

In some embodiments, when it is determined that the correction is needed, the system calibration result may be corrected first, and then the air correction table may be corrected, or the air correction table and the system calibration result may be corrected simultaneously, which is not limited in this embodiment.

For example, when it is determined that the correction processing is required, the system calibration process may be re-performed first. After re-performing the system calibration, new air data collected during system calibration and a corrected system calibration result are acquired. A third air correction table is generated based on daily collected air data and the new air data collected during system calibration. Then image reconstruction may be performed on the scanning data by performing the process 200 based on the corrected system calibration result and the third air correction table. The operations 204-210 may be performed again using the corrected system calibration result and the third air correction table to perform test scanning, and a new test target image (which is referred to as a second test target image) may be obtained. Then the quality of the second test target image may be determined. If the second test target image satisfies the requirement (e.g., the first preset condition), image reconstruction may be performed on the scanning data by performing the process 200 based on the corrected system calibration result and the third air correction table. If the second test target image does not satisfy the requirement (e.g., the first preset condition), the daily air scanning may be re-performed. After daily air scanning is re-performed, new daily collected air data is acquired. A fourth air correction table is generated based on the new daily collected air data and the new air data collected during system calibration. Then image reconstruction may be performed on the scanning data by performing the process 200 based on the fourth air correction table and the corrected system calibration result.

As another example, when it is determined that correction processing is required, the system calibration may be re-performed, and the daily air scanning may be re-performed. New daily collected air data, new air data collected during system calibration, and a corrected system calibration result are acquired. The air correction table may be corrected using the newly collected air data and the new air data collected during system calibration. Then image reconstruction may be performed on the scanning data by performing the process 200 based on the corrected air correction table and the corrected system calibration result.

In some embodiments, in actual clinical application, the process of determining the quality of the image and the correction of data preprocessing and data post-processing may be added on the basis of obtaining the target image, which is not limited in the present disclosure. For example, after the target image is obtained, the processing device may determine whether the target image satisfies a preset condition; in response to determining that the target image does not satisfy the preset condition, the processing device may optimize the data preprocessing and/or data post-processing mode, and obtain, based on the optimized data preprocessing and /or optimized data post-processing, a new target image. The preset condition may include whether the image quality satisfies the requirements.

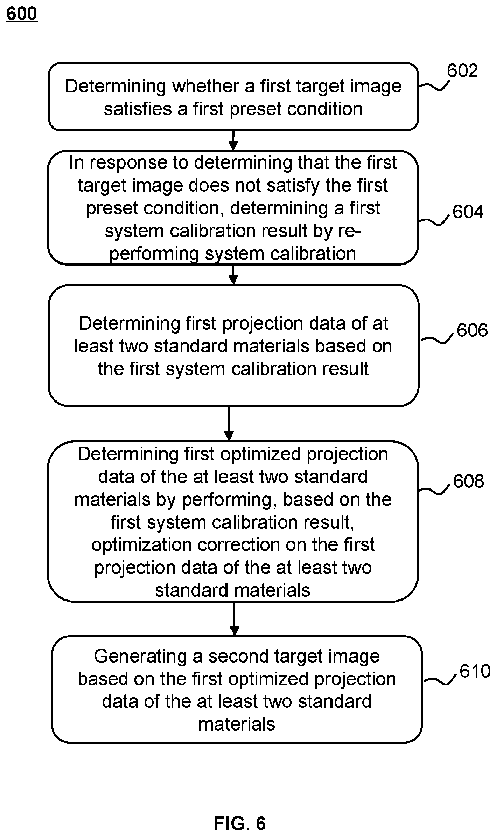

More descriptions regarding determining the image quality may be found in the related descriptions of FIG. 5 and FIG. 6.

According to the method for CT imaging provided in some embodiments of the present disclosure, the air data collected daily is used for correction in the process of material decomposition in the projection domain, and the response model parameter obtained by system calibration is used for basis material decomposition. The quality of the target image generated based on the projection data of the at least two standard materials can also be determined. When the image quality does not satisfy the use requirements, the air correction table and/or the response model parameter are corrected, and the target image is re-acquired, which can effectively improve the quality of the obtained image.

FIG. 3 is a flowchart illustrating an exemplary process of determining an optimization parameter according to some embodiments of the present disclosure. In some embodiments, a process 300 may be performed by a system for CT imaging (e.g., the system for CT imaging 900 in FIG. 9) or a processing device (e.g., the processing device 140). As shown in FIG. 3, the process 300 may include the following operations.

In 302, system calibration data may be obtained. The system calibration data may include the Photon-Counting rate recorded during the system calibration and the corresponding path integral length (also referred to as the actual path integral length). More descriptions regarding the system calibration data may be found in the descriptions in FIG. 2.

In 304, an estimated path integral length may be determined by inputting a Photon-Counting rate of the system calibration data into a response model configured for basis material decomposition.

The estimated path integral length refers to a path integral length determined based on the response model. The response model may be as shown in Formula (2), or may be a machine learning model described above.

In 306, an optimization parameter may be determined based on the estimated path integral length and the actual path integral length.

In some embodiments, the processing device may determine the optimization parameter based on the estimated path integral length and the actual path integral length. For example, the processing device may determine a mapping relationship between the projection data of the at least two standard materials and the optimized projection data of the at least two standard materials based on the estimated path integral length and the actual path integral length, and determine the optimization parameter based on the mapping relationship. More descriptions regarding determining the optimization parameter based on the estimated path integral length and the actual path integral length may be found in the description in FIG. 4.

In some embodiments, the optimization parameter may be determined during the system calibration. Accordingly, the system calibration result may further include the optimization parameter.

In some embodiments, each detector unit may correspond to an optimization parameter. The processing device may determine the optimization parameter corresponding to a detector unit by performing the process 300 using the system calibration data corresponding to the detector unit.

FIG. 4 is a flowchart illustrating an exemplary process of determining an optimization parameter according to some embodiments of the present disclosure. In some embodiments, a process 400 may be performed by (e.g., the system for CT imaging 900 in FIG. 9) or a processing device (e.g., the processing device 140). As shown in FIG. 4, the process 400 may include the following operations.

In 402, a mapping relationship between projection data of at least two standard materials and optimized projection data of the at least two standard materials may be determined based on an estimated path integral length and an actual path integral length.

In 404, the mapping relationship may be determined as an optimization parameter.

For ease of understanding, the overall process shown in the following embodiment explains how to determine the optimization parameter.

In S1, an estimated path integral length and an actual path integral length in system calibration data may be obtained. The system calibration data may include a Photon-Counting rate detected by each detection unit and a corresponding path integral length recorded during the system calibration. The estimated path integral length refers to an output value obtained by inputting the Photon-Counting rate in the system calibration data into a determined response model (e.g., the above Formula (2) or a machine learning model). The actual path integral length refers to a path integral length corresponding to the input Photon-Counting rate in the system calibration data. More descriptions may be found in the operation 302 and the operation 304 above.

In S2, for each detection unit, the optimization parameter corresponding to the detection unit may be determined according to the estimated path integral length and the actual path integral length corresponding to the detection unit.

In some embodiments, the processing device may determine the mapping relationship between the projection data of the at least two standard materials and the optimized projection data of the at least two standard materials by the following Formula (6): $\left({L}_{1}^{′}, {L}_{2}^{′}\right) = S \left({L}_{10}, {L}_{20}, {L}_{1}, {L}_{2}\right)$ where ( ${L}_{1}^{′} , {L}_{2}^{′}$) denotes the optimized projection data of the at least two standard materials, *L*₁₀, *L*₂₀ denote a thickness of the system calibration material, *L*₁, *L*₂ denote the projection data of the at least two standard materials, and *S*(*) denotes the mapping relationship between the projection data of the at least two standard materials and the optimized projection data of the at least two standard materials. For example, *S*(*) may be determined based on the estimated path integral length and the actual path integral length using interpolation (e.g., linear interpolation), machine learning, and other processes.

In some embodiments, after the mapping relationship is determined, the mapping relationship may be directly used as the optimization parameter.

When the optimization correction is performed, the processing device may obtain the optimized projection data of the at least two standard materials by multiplying each element of the projection data of the at least two standard materials by the corresponding optimization parameter, or obtain, for each detection unit, the optimized projection data of the at least two standard materials corresponding to the detection unit by inputting the projection data of the at least two standard materials corresponding to the detection unit into Formula (6). For example, referring to Formula (2) and Formula (6), the optimized projection data of the at least two standard materials ( ${L}_{1}^{′} , {L}_{2}^{′}$) corresponding to the detection unit may be determined according to the following Formula (7): $\left({L}_{1}^{′}, {L}_{2}^{′}\right) = S \left[{f}^{- 1}\left({y}_{b}\right)\right]$

Therefore, the optimization correction may also be understood as further correction of the response model parameter determined during the system calibration.

In some embodiments, the processing device may first obtain the system calibration data; for each detection unit, the processing device may perform model training based on the system calibration data using the estimated path integral length as an input of model training and the actual path integral length as label data to obtain an optimized correction model (i.e., a trained machine learning model, such as a deep neural network model, etc.). The optimized correction model reflects the mapping relationship between the optimized projection data of the at least two standard materials and the projection data of the at least two standard materials, and the parameter of the optimized correction model may be regarded as the mapping relationship (i.e., the optimization parameter).

When the optimization correction is performed, for each detection unit, the projection data of the at least two standard materials corresponding to the detection unit may be input into the corresponding trained optimization correction model, and the optimized projection data of the at least two standard materials corresponding to the detection unit may be output.

FIG. 5 is a flowchart illustrating an exemplary process of generating a first target image according to some embodiments of the present disclosure. In some embodiments, a process 500 may be performed by (e.g., the system for CT imaging 900 in FIG. 9) or a processing device (e.g., the processing device 140). As shown in FIG. 5, the process 500 may include the following operations.

In 502, whether a target image satisfies a first preset condition may be determined.

The first preset condition may include whether an image quality of the target image satisfies preset requirements, for example, whether an overall image quality satisfies the preset requirements, or whether a certain quality index (e.g., whether there are artifacts, image resolution, image contrast, etc.) of the target image satisfies the preset requirements.

In some embodiments, whether the target image satisfies the first preset condition may be determined through a manual mode or an algorithm. For example, whether the target image satisfies the first preset condition may be determined using a trained image quality determination model. The image quality determination model may be a trained machine learning model, such as a deep neural network, etc., which is not limited in this embodiment.

In response to determining that the target image satisfies the first preset condition, the target image may be output as a final reconstructed image.

In 504, in response to determining that the target image does not satisfy the first preset condition, an air correction table may be updated.

In response to determining that the target image does not satisfy the first preset condition, it means that the quality of the target image can be further improved. Correspondingly, in response to determining that the target image does not satisfy the first preset condition, it also means that optimization is required in the current process of the method for CT imaging. Given that the complexity of air correction is much less than system calibration, in this embodiment, the air correction table may be updated first to obtain more accurate data for imaging.

The air correction table may be updated by re-performing air scanning to obtain new current air data and constructing the air correction table based on the new current air data.

It should be noted that the air scanning is not required for every imaging. For example, the air scanning may be performed and the air correction table may be updated once a week. Accordingly, the air correction may not be accurate enough for the current scanning data. In this case, the latest air data for the current scanning environment may be obtained and the air correction table may be updated.

More descriptions regarding constructing the air correction table may be found in the related description of the operation 204, which are not repeated here.

In 506, first data after air correction may be determined by performing, based on an updated air correction table (e.g., a first air correction table), air correction on scanning data.

More descriptions regarding the operation 506 may be found in the related descriptions of air correction in the operation 204, and the difference is that different air correction tables are used.

In 508, a first target image may be generated based on the first data after air correction.

In some embodiments, the processing device may generate the first target image by performing the operations 206-210 of FIG. 2 of the present disclosure based on the first data after air correction. The first target image may be directly used as the final reconstructed image, or the process 600 may be performed based on the first target image.

FIG. 6 is a flowchart illustrating an exemplary process of generating a second target image according to some embodiments of the present disclosure. In some embodiments, a process 600 may be performed by (e.g., the system for CT imaging 900 in FIG. 9) or a processing device (e.g., the processing device 140). As shown in FIG. 6, the process 600 may include the following operations.

In 602, whether a first target image satisfies a first preset condition may be determined. In response to determining that the first target image satisfies the first preset condition, the first target image may be used as a final reconstructed image.

More descriptions regarding the 602 may be found in the related descriptions of the operation 502.

In 604, in response to determining that the first target image does not satisfy the first preset condition, a first system calibration result may be determined by re-performing system calibration. The first system calibration result may include at least one of a system calibration material, system calibration data, a response model parameter, an optimization parameter, etc.

In 606, first projection data of at least two standard materials may be determined based on the first system calibration result.

In the process 500, daily air scanning is re-performed, and new current air data is acquired. The first air correction table is generated based on the new current air data and original reference air data. In the process 600, after re-performing the system calibration, new reference air data and the first system calibration result are acquired. A second air correction table is generated based on the new reference air data and the new current air data. Second data after air correction is determined by performing, based on the second air correction table, air correction on the scanning data. The first projection data of the at least two standard materials may be determined by performing, based on the first system calibration result, basis material decomposition on the second data after air correction.

In 608, first optimized projection data of the at least two standard materials may be determined by performing, based on the first system calibration result, optimization correction on the first projection data of the at least two standard materials.

In 610, a second target image may be generated based on the first optimized projection data of the at least two standard materials. The second target image may be used as the final reconstructed image.

It should be noted that when the system calibration is re-performed, the air data corresponding to the system calibration (reference air data) updates accordingly. Accordingly, the air correction table is updated based on the air data collected during re-performing the system calibration.

Therefore, in some embodiments, the process of generating the second target image may include: in response to determining that the image quality of the target image does not satisfy the preset requirements, re-performing daily air scanning and obtaining new current air data; determining a first air correction table based on the new current air data and original reference air data; performing operations 204-210 based on the first air correction table, and generating the first target image; determining whether the image quality of the first target image satisfies the preset requirements; in response to determining that the image quality of the first target image does not satisfy the preset requirements, re-performing the system calibration and obtaining new reference air data and a new system calibration result; determining a second air correction table based on the new reference air data acquired by re-performing the system calibration and the new current air data; and generating the second target image by re-performing, based on the second air correction table and the new system calibration result, the process of FIG. 2 (e.g., operations 204-210, or operations 606-610).

More descriptions regarding the operations 604-610 may be found in the related descriptions of the operations 204-206 in FIG. 2, and the difference is that the system calibration in the operations 204-206 is performed in advance, while the real-time system calibration is performed based on the current scanning environment in the operations 604-610. More descriptions regarding the system calibration in the operations 604-610 may be found in the related descriptions in the operations 204-206.

In some embodiments, in response to determining that the target image does not satisfy the first preset condition, the system calibration may be re-performed first, and the daily air scanning may be re-performed. For example, in response to determining that the target image does not satisfy the first preset condition, the system calibration may be re-performed to obtain the first system calibration result and new reference air data. A third air correction table is generated based on original current air data and the new reference air data. The operations 204-210 in FIG. 2 may be performed based on the first system calibration result and the third air correction table to generate a third target image. The third target image may be directly used as the final reconstructed image, or whether the third target image satisfy the first preset condition may be determined. In response to determining that the third target image satisfy the first preset condition, the third target image may be used as the final reconstructed image. In response to determining that the third target image does not satisfy the first preset condition, the daily air scanning may be re-performed. After daily air scanning is re-performed, new current air data is acquired. A fourth air correction table is generated based on the new current air data and the new reference air data. The operations 204-210 in FIG. 2 in the present disclosure may be performed based on the fourth air correction table and the first system calibration result to obtain a fourth target image. The fourth target image may be used as the final reconstructed image.

In some embodiments, in response to determining that the target image does not satisfy the first preset condition, the system calibration may be re-performed and the daily air scanning may be re-performed simultaneously. For example, in response to determining that the target image does not satisfy the first preset condition, the system calibration may be re-performed to obtain the first system calibration result and new reference air data, and the daily air scanning may be re-performed to obtain new current air data. An updated air correction table may be determined based on the new reference air data and the new current air data. The operations 204-210 in FIG. 2 in the present disclosure may be performed based on the updated air correction table and the first system calibration result to obtain a fifth target image. The fifth target image may be used as the final reconstructed image.

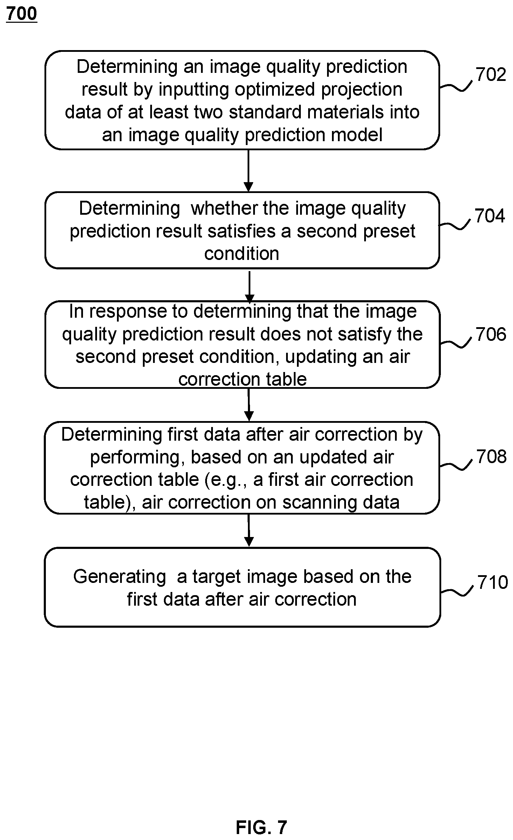

FIG. 7 is a flowchart illustrating an exemplary process of generating a target image according to some embodiments of the present disclosure. In some embodiments, a process 700 may be performed by (e.g., the system for CT imaging 900 in FIG. 9) or a processing device (e.g., the processing device 140). As shown in FIG. 7, the process 700 may include the following operations.

In 702, an image quality prediction result may be determined by inputting optimized projection data of at least two standard materials into an image quality prediction model.

The image quality prediction model may be a machine learning model that is trained to predict image quality, such as a deep neural network model, etc.

The image quality prediction result refers to a prediction result output by the image quality prediction model for the image generated based on the optimized projection data of the at least two standard materials. For example, the image quality prediction result may be expressed in the form of a score, such as 1-10, which respectively represent different image quality levels. The closer the score is to 10, the better the predicted image quality. As another example, the image quality prediction result may be expressed in the form of a vector, and an element at each position of the vector may represent a score of a certain index of the image quality, such as resolution, degree of artifacts, contrast, etc. A size of each element of the vector may correspond to a different score (e.g., expressed in a numerical form). Accordingly, an overall score of the image quality may be an average of the scores of the elements of the vector.

In some embodiments, the image quality prediction model may be obtained by training an initial deep neural network model with training data. For example, sample optimized projection data of at least two standard materials in historical scans may be used as training samples. A sample image may be generated based on the sample optimized projection data of the at least two standard materials. Image quality may be determined based on the sample image. A corresponding image quality score of the sample image may be obtained, and the image quality score may be used as a label for model training. After the model is trained by a model training process such as gradient descent, the image quality prediction model may be obtained.

In 704, whether the image quality prediction result satisfies a second preset condition may be determined.

The second preset condition may include that the image quality prediction result does not satisfy a preset requirement. For example, the overall score of the image quality does not satisfy a preset score threshold, etc. In response to determining that the image quality prediction result satisfies the second preset condition, the target image may be generated based on the optimized projection data of the at least two standard materials.

In 706, in response to determining that the image quality prediction result does not satisfy the second preset condition, the air correction table may be updated.

In response to determining that the image quality prediction result does not satisfy the second preset condition, it means that the quality of the image obtained by a subsequent process (e.g., an image reconstruction process) based on the current optimized projection data of the at least two standard materials may not satisfy an image quality requirement (e.g., the first preset condition). Therefore, in order to save the overall process time, the air correction table may be directly updated in response to determining that the image quality prediction result does not satisfy the second preset condition.

More descriptions regarding updating the air correction table may be found in the related descriptions of FIG. 5.

In 708, first data after air correction may be determined by performing, based on an updated air correction table (e.g., a first air correction table), air correction on the scanning data.

In 710, a target image may be generated based on the first data after air correction.

In some embodiments, since the process of generating the target image based on the first data after air correction is similar to the process described above, the process is briefly described below. For example, the process of generating the target image based on the first data after air correction may include: generating first projection data of at least two standard materials based on the first data after air correction; and performing optimization correction based on the first projection data of the at least two standard materials to obtain first optimized projection data of the at least two standard materials. The processing device may directly generate the target image based on the first optimized projection data of the at least two standard materials. The processing device may also determine whether a predicted image quality corresponding to the first optimized projection data of the at least two standard materials satisfies the second preset condition. In response to determining that the predicted image quality corresponding to the first optimized projection data of the at least two standard materials satisfies the second preset condition, the target image may be generated based on the first optimized projection data of the at least two standard materials. In response to determining that the predicted image quality corresponding to the first optimized projection data of the at least two standard materials does not satisfy the second preset condition, the system calibration may be re-performed. In the process 700, daily air scanning is re-performed, and new current air data is acquired. The first air correction table is generated based on the new current air data and original reference air data. After re-performing the system calibration, new reference air data and a first system calibration result are acquired. A second air correction table is generated based on the new reference air data and the new current air data. The operations 204-208 in FIG. 2 in the present disclosure may be performed based on the second air correction table and the first system calibration result obtained by re-performing the system calibration to obtain second optimized projection data of at least two standard materials. The target image may be generated based on the second optimized projection data of the at least two standard materials.

More descriptions regarding the operation 708 and the operation 710 may be found in the related descriptions of the operations 204-210 in FIG. 2.

In some embodiments, in response to determining that an image quality prediction result of the optimized projection data of the at least two standard materials does not satisfy the second preset condition, the system calibration may be re-performed first, and the daily air scanning may be re-performed. For example, in response to determining that the image quality prediction result of the optimized projection data of the at least two standard materials does not satisfy the second preset condition, the system calibration may be re-performed to obtain the first system calibration result and new reference air data. A third air correction table is generated based on original current air data and the new reference air data. The operations 204-208 of FIG. 2 may be performed based on the first system calibration result and the third air correction table to generate third optimized projection data of at least two standard materials. The processing device may directly generate the target image based on the third optimized projection data of the at least two standard materials. The processing device may also determine whether the predicted image quality corresponding to the third optimized projection data of the at least two standard materials satisfies the second preset condition. In response to determining that the predicted image quality corresponding to the third optimized projection data of the at least two standard materials satisfies the second preset condition, the target image may be generated according to the third optimized projection data of the at least two standard materials. In response to determining that the predicted image quality corresponding to the third optimized projection data of the at least two standard materials does not satisfy the second preset condition, the daily air scanning may be re-performed. After daily air scanning is re-performed, new current air data is acquired. A fourth air correction table is generated based on the new current air data and the new reference air data. The operations 204-208 of FIG. 2 in the present disclosure may be performed based on the fourth air correction table and the first system calibration result to obtain fourth optimized projection data of the at least two standard materials. The target image may be generated based on the fourth optimized projection data of the at least two standard materials.

In some embodiments, in response to determining that the image quality prediction result does not satisfy the second preset condition, the system calibration may be re-performed and the daily air scanning may be re-performed simultaneously. For example, in response to the image quality prediction result does not satisfy the second preset condition, the system calibration may be re-performed to obtain the first system calibration result and new reference air data, and the daily air scanning may be re-performed to obtain new current air data. An updated air correction table may be determined based on the new reference air data and the new current air data. The operations 204-210 of FIG. 2 in the present disclosure may be performed based on the updated air correction table and the first system calibration result to obtain the target image.

Since the first preset condition and the second preset condition are configured to evaluate the image quality, the first preset condition and the second preset condition may be the same or different.

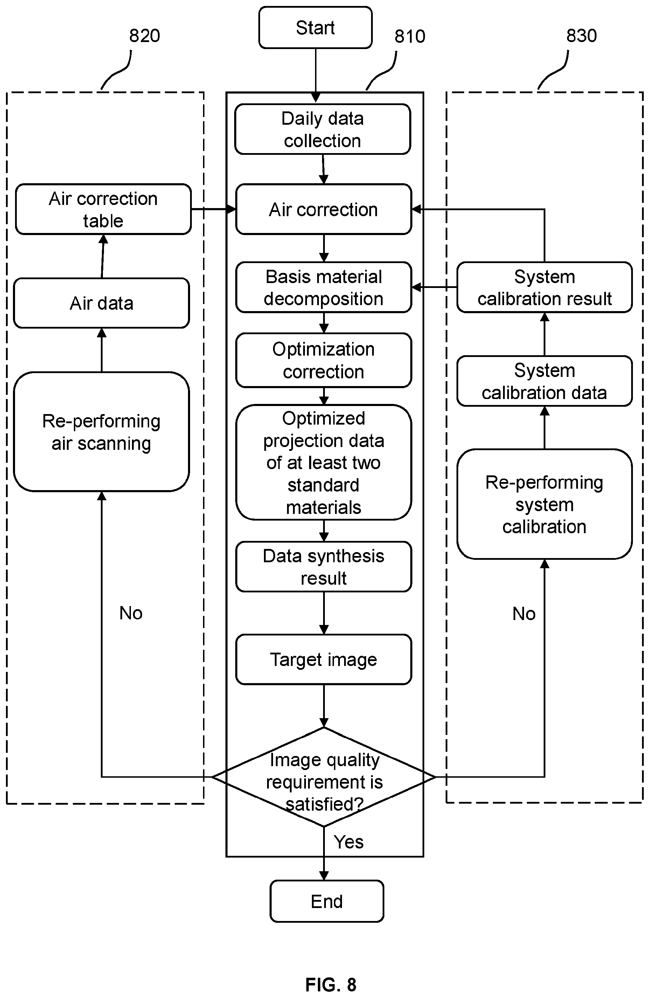

FIG. 8 is a flowchart illustrating an exemplary method for CT imaging according to some embodiments of the present disclosure.

As shown in FIG. 8, the process of the method for CT imaging may include a sub-operation 810, a sub-operation 820, and a sub-operation 830.

In the sub-operation 810, a process of daily data collection may include obtaining scanning data of a target subject (i.e., data collection during clinical application), and obtaining projection data of a phantom (i.e., a system test before routine clinical application). After the scanning data is obtained, an air calibration table acquired in advance may be used for air correction (not shown in the figure).

A process of basis material decomposition may include performing material decomposition using a pre-acquired response model parameter (not shown in the figure).

Then optimized projection data of the at least two standard materials may be determined by performing optimization correction on the projection data of the at least two standard materials; a data synthesis result may be determined by performing data synthesis based on the optimized projection data of the at least two standard materials; and a target image may be generated based on the data synthesis result.

In response to determining that the image quality of the target image does not satisfy the first preset condition, the processing device may perform sub-operation 820 and/or sub-operation 830.

In the sub-operation 820, the processing device may re-perform air scanning, and obtain new current air data corresponding to the scanning data. Then the air correction table may be updated based on the new current air data. The processing device may re-perform the sub-operation 810 based on the updated air correction table to generate a new target image.

In the sub-operation 830, the processing device may re-perform system calibration, and obtain a new system calibration result. The processing device may re-perform the sub-operation 810 based on the new system calibration result to generate a new target image.

It should be noted that, one of the sub-operation 820 and the sub-operation 830 may be selected for execution, or both of the sub-operation 820 and the sub-operation 830 may be executed, which is not limited in this embodiment .

More descriptions regarding each operation of FIG. 8 may be found in the related descriptions of FIGs. 2-7, which are not repeated here.

It should be noted that the above descriptions of each process are only for illustration and explanation, and do not limit the scope of application of the present disclosure. For those skilled in the art, various modifications and changes can be made to each process under the guidance of the present disclosure. However, these modifications and changes are still within the scope of the present disclosure. For example, a storage operation may be added between each process, etc. As another example, some operations in each process may be omitted in some embodiments. For example, in the process shown in FIG. 2, only the air correction (i.e., the operation 204) may be performed and the optimization correction (i.e., the operation 208) may be omitted, or only the optimization correction (i.e., the operation 208) may be performed and the air correction (i.e., the operation 204) may be omitted.

FIG. 9 is a block diagram illustrating an exemplary system for CT imaging according to some embodiments of the present disclosure.

The system for CT imaging may include a scanning data acquisition module 910, a correction module 920, a decomposition module 930, an optimization module 940, and an imaging module 950. The scanning data acquisition module 910 may be configured to obtain scanning data of a target subject. The scanning data may be acquired by scanning the target subject using a CT device. The correction module 920 may be configured to determine data after air correction by performing air correction on the scanning data using an air correction table. The decomposition module 930 may be configured to determine projection data of at least two standard materials by performing basis material decomposition on the data after air correction. The optimization module 940 may be configured to determine optimized projection data of the at least two standard materials by performing optimization correction on the projection data of the at least two standard materials. The imaging module 950 may be configured to generate a target image based on the optimized projection data of the at least two standard materials.

The present disclosure provides a system for CT imaging including one or more storage devices (e.g., the storage device 150) and one or more processors (e.g., the processing device 140) configured to communicate with the one or more storage devices. The one or more storage devices may include a set of instructions. When the one or more processors executing the set of instructions, the one or more processors may be directed to perform the method for CT imaging (e.g., the processes illustrated in FIGs. 2-8) illustrated above.

The present disclosure provides a non-transitory computer readable medium, comprising at least one set of instructions for CT imaging, wherein when executed by one or more processors (e.g., the processing device 140) of a computing device, the at least one set of instructions causes the computing device to perform the method for CT imaging (e.g., the processes illustrated in FIGs. 2-8) illustrated above.

It should be noted that the above description of the system and its modules is only for convenience of description and cannot limit the present disclosure to the scope of the embodiments. It is understood that for those skilled in the art, after understanding the principle of the system, it is possible to arbitrarily combine the various modules without deviating from this principle, or to form a subsystem connected with other modules. In some embodiments, the

Scanning data acquisition module 910, Correction module 920, Decomposition module 930, Optimization module 940 and Imaging module 950 disclosed in FIG. 9 can be different modules in a system, or a module can realize the functions of two or more modules mentioned above. For example, each module can share a storage module, and each module can also have its own storage module. Such variations are all within the protection scope of the present disclosure.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure and are within the spirit and scope of the exemplary embodiments of this disclosure.

Moreover, certain terminology has been used to describe embodiments of the present disclosure. For example, the terms "one embodiment," "an embodiment," and "some embodiments" mean that a particular feature, structure, or feature described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures, or features may be combined as suitable in one or more embodiments of the present disclosure.

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose and that the appended claims are not limited to the disclosed embodiments, but, on the contrary, are intended to cover modifications and equivalent arrangements that are within the spirit and scope of the disclosed embodiments. For example, although the implementation of various parts described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. This method of disclosure, however, is not to be interpreted as reflecting an intention that the claimed subject matter requires more features than are expressly recited in each claim. Rather, claimed subject matter may lie in less than all features of a single foregoing disclosed embodiment.

In some embodiments, numbers describing the number of ingredients and attributes are used. It should be understood that such numbers used for the description of the embodiments use the modifier "about", "approximately", or "substantially" in some examples. Unless otherwise stated, "about", "approximately", or "substantially" indicates that the number is allowed to vary by ±20%. Correspondingly, in some embodiments, the numerical parameters used in the description and claims are approximate values, and the approximate values may be changed according to the required features of individual embodiments. In some embodiments, the numerical parameters should consider the prescribed effective digits and adopt the method of general digit retention. Although the numerical ranges and parameters used to confirm the breadth of the range in some embodiments of the present disclosure are approximate values, in specific embodiments, settings of such numerical values are as accurate as possible within a feasible range.

For each patent, patent application, patent application publication, or other materials cited in the present disclosure, such as articles, books, specifications, publications, documents, or the like. The application history documents that are inconsistent or conflict with the content of the present disclosure are excluded, and the documents that restrict the broadest scope of the claims of the present disclosure (currently or later attached to the present disclosure) are also excluded. It should be noted that if there is any inconsistency or conflict between the description, definition, and/or use of terms in the auxiliary materials of the present disclosure and the content of the present disclosure, the description, definition, and/or use of terms in the present disclosure is subject to the present disclosure.

Finally, it should be understood that the embodiments described in the present disclosure are only used to illustrate the principles of the embodiments of the present disclosure. Other variations may also fall within the scope of the present disclosure. Therefore, as an example and not a limitation, alternative configurations of the embodiments of the present disclosure may be regarded as consistent with the teaching of the present disclosure. Accordingly, the embodiments of the present disclosure are not limited to the embodiments introduced and described in the present disclosure explicitly.

## Claims

1. A method for computed tomography (CT) imaging, comprising:
obtaining scanning data of a target subject, the scanning data being acquired by scanning the target subject using a CT device;
determining data after air correction by performing air correction on the scanning data using an air correction table;
determining projection data of at least two standard materials by performing basis material decomposition on the data after air correction;
generating a target image based on the projection data of the at least two standard materials,
**characterised in that** generating a target image includes:
determining optimized projection data of the at least two standard materials by performing optimization correction on the projection data of the at least two standard materials, including:
obtaining an optimization parameter that is determined based on system calibration data; and
determining the optimized projection data of the at least two standard materials by performing, based on the optimization parameter, the optimization correction on the projection data of the at least two standard materials; and
generating the target image based on the optimized projection data of the at least two standard materials.

2. The method of claim 1, wherein the optimization parameter is determined by:
obtaining the system calibration data by scanning at least two system calibration materials, the system calibration data including a Photon-Counting rate detected by a detector of the CT device and an actual path integral length of the at least two system calibration materials corresponding to the Photon-Counting rate;
determining an estimated path integral length by inputting the Photon-Counting rate of the system calibration data into a response model configured for the basis material decomposition; and
determining the optimization parameter based on the estimated path integral length and the actual path integral length.

3. The method of claim 2, wherein the determining the optimization parameter based on the estimated path integral length and the actual path integral length includes:
determining a mapping relationship between the optimized projection data of the at least two standard materials and the projection data of the at least two standard materials based on the estimated path integral length and the actual path integral length; and
determining the mapping relationship as the optimization parameter.

4. The method of claim 2 or claim 3, wherein the determining the optimization parameter based on the estimated path integral length and the actual path integral length includes:
generating an optimization model by performing, based on the estimated path integral length and the actual path integral length, a model training process, the actual path integral length being used as a label of the estimated path integral length.

5. The method of any one of claims 1-4, further comprising:
determining whether the target image satisfies a first preset condition;
in response to determining that the target image does not satisfy the first preset condition, updating the air correction table;
determining first data after air correction by performing, based on an updated air correction table, air correction on the scanning data; and
generating a first target image based on the first data after air correction.

6. The method of any one of claims 1-5, wherein the updating the air correction table includes:
obtaining current air data corresponding to the scanning data;
obtaining reference air data corresponding to system calibration; and
determining the updated air correction table based on the current air data and the reference air data.

7. The method of claim 5 or claim 6, further comprising:
determining whether the first target image satisfies the first preset condition;
in response to determining that the first target image does not satisfy the first preset condition, determining a first system calibration result by re-performing system calibration; and
generating a second target image based on the first system calibration result.

8. The method of any one of claims 1-6, wherein the generating a target image based on the optimized projection data of the at least two standard materials includes:
determining a first image quality prediction result by inputting the optimized projection data of the at least two standard materials into an image quality prediction model;
determining whether the first image quality prediction result satisfies a second preset condition;
in response to determining that the image quality prediction result does not satisfy the second preset condition, updating the air correction table;
determining first data after air correction by performing, based on an updated air correction table, air correction on the scanning data; and
generating the target image based on the first data after air correction.

9. The method of claim 8, wherein the generating the target image based on the first data after air correction includes:
determining first projection data of at least two standard materials by performing basis material decomposition on the first data after air correction;
determining first optimized projection data of the at least two standard materials by performing optimization correction on the first projection data of the at least two standard materials;
determining a second image quality prediction result by inputting the first optimized projection data of the at least two standard materials into the image quality prediction model;
determining whether the second image quality prediction result satisfy the second preset condition;
in response to determining that the second image quality prediction result does not satisfy the second preset condition, determining a first system calibration result by re-performing system calibration; and
generating the target image based on the first system calibration result.

10. A system for computed tomography (CT) imaging, comprising:
a scanning data acquisition module (910) configured to obtain scanning data of a target subject, the scanning data being acquired by scanning the target subject using a CT device;
a correction module (920) configured to determine data after air correction by performing air correction on the scanning data using an air correction table;
a decomposition module (930) configured to determine projection data of at least two standard materials by performing basis material decomposition on the data after air correction;
an optimization module (940) configured to determine optimized projection data of the at least two standard materials by performing optimization correction on the projection data of the at least two standard materials; and
an imaging module (950) configured to generate a target image based on the optimized projection data of the at least two standard materials,
**characterised in that** generating a target image includes:
determining optimized projection data of the at least two standard materials by performing optimization correction on the projection data of the at least two standard materials, including:
obtaining an optimization parameter that is determined based on system calibration data; and
determining the optimized projection data of the at least two standard materials by performing, based on the optimization parameter, the optimization correction on the projection data of the at least two standard materials; and
generating the target image based on the optimized projection data of the at least two standard materials.

11. The system of claim 10, wherein the determining optimized projection data of the at least two standard materials by performing optimization correction on the projection data of the at least two standard materials includes:
obtaining an optimization parameter that is determined based on system calibration data; and
determining the optimized projection data of the at least two standard materials by performing, based on the optimization parameter, the optimization correction on the projection data of the at least two standard materials.

12. The system of claim 10 or claim 11, wherein the air correction table is obtained by:
obtaining current air data corresponding to the scanning data;
obtaining reference air data corresponding to system calibration; and
determining the air correction table based on the current air data and the reference air data.

13. A non-transitory computer readable medium, comprising at least one set of instructions, wherein when executed by one or more processors of a computing device, the at least one set of instructions causes the computing device to perform the CT imaging method of any one of claims 1-9.

## Patentansprüche

1. Verfahren für Computertomographie-(CT)-Bildgebung, umfassend:
Erhalten von Scandaten eines Zielsubjekts, wobei die Scandaten durch Scannen des Zielsubjekts unter Verwendung einer CT-Vorrichtung erfasst werden;
Bestimmen von Daten nach Luftkorrektur durch Durchführen von Luftkorrektur an den Scandaten unter Verwendung einer Luftkorrekturtabelle;
Bestimmen von Projektionsdaten von mindestens zwei Standardmaterialien durch Durchführen von Basismaterialzerlegung an den Daten nach Luftkorrektur;
Erzeugen eines Zielbildes basierend auf den Projektionsdaten der mindestens zwei Standardmaterialien,
**dadurch gekennzeichnet, dass** Erzeugen eines Zielbildes Folgendes einschließt:
Bestimmen optimierter Projektionsdaten der mindestens zwei Standardmaterialien durch Durchführen von Optimierungskorrektur an den Projektionsdaten der mindestens zwei Standardmaterialien, einschließend:
Erhalten eines Optimierungsparameters, der basierend auf Systemkalibrierungsdaten bestimmt wird; und
Bestimmen der optimierten Projektionsdaten der mindestens zwei Standardmaterialien durch Durchführen, basierend auf dem Optimierungsparameter, der Optimierungskorrektur an den Projektionsdaten der mindestens zwei Standardmaterialien; und
Erzeugen des Zielbildes basierend auf den optimierten Projektionsdaten der mindestens zwei Standardmaterialien.

2. Verfahren nach Anspruch 1, wobei der Optimierungsparameter bestimmt wird durch:
Erhalten der Systemkalibrierungsdaten durch Scannen von mindestens zwei Systemkalibrierungsmaterialien, wobei die Systemkalibrierungsdaten eine von einem Detektor der CT-Vorrichtung detektierte Photonenzählrate und eine der Photonenzählrate entsprechende tatsächliche Pfadintegrallänge der mindestens zwei Systemkalibrierungsmaterialien einschließen;
Bestimmen einer geschätzten Pfadintegrallänge durch Eingeben der Photonenzählrate der Systemkalibrierungsdaten in ein für die Basismaterialzerlegung konfiguriertes Antwortmodell; und
Bestimmen des Optimierungsparameters basierend auf der geschätzten Pfadintegrallänge und der tatsächlichen Pfadintegrallänge.

3. Verfahren nach Anspruch 2, wobei das Bestimmen des Optimierungsparameters basierend auf der geschätzten Pfadintegrallänge und der tatsächlichen Pfadintegrallänge Folgendes einschließt:
Bestimmen einer Abbildungsbeziehung zwischen den optimierten Projektionsdaten der mindestens zwei Standardmaterialien und den Projektionsdaten der mindestens zwei Standardmaterialien basierend auf der geschätzten Pfadintegrallänge und der tatsächlichen Pfadintegrallänge; und
Bestimmen der Abbildungsbeziehung als den Optimierungsparameter.

4. Verfahren nach Anspruch 2 oder 3, wobei das Bestimmen des Optimierungsparameters basierend auf der geschätzten Pfadintegrallänge und der tatsächlichen Pfadintegrallänge Folgendes einschließt:
Erzeugen eines Optimierungsmodells durch Durchführen, basierend auf der geschätzten Pfadintegrallänge und der tatsächlichen Pfadintegrallänge, eines Modelltrainingsprozesses, wobei die tatsächliche Pfadintegrallänge als Label für die geschätzte Pfadintegrallänge verwendet wird.

5. Verfahren nach einem der Ansprüche 1-4, weiter umfassend:
Bestimmen, ob das Zielbild eine erste voreingestellte Bedingung erfüllt;
als Reaktion auf Bestimmen, dass das Zielbild die erste voreingestellte Bedingung nicht erfüllt, Aktualisieren der Luftkorrekturtabelle;
Bestimmen von ersten Daten nach Luftkorrektur durch Durchführen, basierend auf einer aktualisierten Luftkorrekturtabelle, von Luftkorrektur an den Scandaten; und
Erzeugen eines ersten Zielbildes basierend auf den ersten Daten nach Luftkorrektur.

6. Verfahren nach einem der Ansprüche 1-5, wobei das Aktualisieren der Luftkorrekturtabelle Folgendes einschließt:
Erhalten von aktuellen Luftdaten, die den Scandaten entsprechen;
Bestimmen von Referenzluftdaten, die der Systemkalibrierung entsprechen; und
Bestimmen der aktualisierten Luftkorrekturtabelle basierend auf den aktuellen Luftdaten und den Referenzluftdaten.

7. Verfahren nach Anspruch 5 oder Anspruch 6, weiter umfassend:
Bestimmen, ob das erste Zielbild die erste voreingestellte Bedingung erfüllt;
als Reaktion auf Bestimmen, dass das erste Zielbild die erste voreingestellte Bedingung nicht erfüllt, Bestimmen eines ersten Systemkalibrierungsergebnisses durch erneutes Durchführen von Systemkalibrierung; und
Erzeugen eines zweiten Zielbildes basierend auf dem ersten Systemkalibrierungsergebnis.

8. Verfahren nach einem der Ansprüche 1-6, wobei das Erzeugen eines Zielbildes basierend auf den optimierten Projektionsdaten der mindestens zwei Standardmaterialien Folgendes einschließt:
Bestimmen eines ersten Bildqualitätsvorhersageergebnisses durch Eingeben der optimierten Projektionsdaten der mindestens zwei Standardmaterialien in ein Bildqualitätsvorhersagemodell;
Bestimmen, ob das erste Bildqualitätsvorhersageergebnis eine zweite voreingestellte Bedingung erfüllt;
als Reaktion auf Bestimmen, dass das Bildqualitätsvorhersageergebnis die zweite voreingestellte Bedingung nicht erfüllt, Aktualisieren der Luftkorrekturtabelle;
Bestimmen von ersten Daten nach Luftkorrektur durch Durchführen, basierend auf einer aktualisierten Luftkorrekturtabelle, von Luftkorrektur an den Scandaten; und
Erzeugen des Zielbildes basierend auf den ersten Daten nach Luftkorrektur.

9. Verfahren nach Anspruch 8, wobei das Erzeugen des Zielbildes basierend auf den ersten Daten nach Luftkorrektur Folgendes einschließt:
Bestimmen von ersten Projektionsdaten von mindestens zwei Standardmaterialien durch Durchführen von Basismaterialzerlegung an den ersten Daten nach Luftkorrektur;
Bestimmen der ersten optimierten Projektionsdaten der mindestens zwei Standardmaterialien durch Durchführen von Optimierungskorrektur an den ersten Projektionsdaten der mindestens zwei Standardmaterialien;
Bestimmen eines zweiten Bildqualitätsvorhersageergebnisses durch Eingeben der ersten optimierten Projektionsdaten der mindestens zwei Standardmaterialien in das Bildqualitätsvorhersagemodell;
Bestimmen, ob das zweite Bildqualitätsvorhersageergebnis die zweite voreingestellte Bedingung erfüllt;
als Reaktion auf Bestimmen, dass das zweite Bildqualitätsvorhersageergebnis die zweite voreingestellte Bedingung nicht erfüllt, Bestimmen eines ersten Systemkalibrierungsergebnisses durch erneutes Durchführen von Systemkalibrierung; und
Erzeugen des Zielbildes basierend auf dem ersten Systemkalibrierungsergebnis.

10. System für Computertomographie-(CT)-Bildgebung, umfassend:
ein Scandatenerfassungsmodul (910), das so konfiguriert ist, dass es Scandaten eines Zielsubjekts erhält, wobei die Scandaten durch Scannen des Zielsubjekts unter Verwendung einer CT-Vorrichtung erfasst werden;
ein Korrekturmodul (920), das so konfiguriert ist, dass es Daten nach Luftkorrektur bestimmt durch Durchführen von Luftkorrektur an den Scandaten unter Verwendung einer Luftkorrekturtabelle;
ein Zerlegungsmodul (930), das so konfiguriert ist, dass es Projektionsdaten von mindestens zwei Standardmaterialien durch Durchführen von Basismaterialzerlegung an den Daten nach Luftkorrektur bestimmt;
ein Optimierungsmodul (940), das so konfiguriert ist, dass es optimierte Projektionsdaten der mindestens zwei Standardmaterialien durch Durchführen von Optimierungskorrektur an den Projektionsdaten der mindestens zwei Standardmaterialien bestimmt; und
ein Bildgebungsmodul (950), das so konfiguriert ist, dass es basierend auf den optimierten Projektionsdaten der mindestens zwei Standardmaterialien ein Zielbild erzeugt,
**dadurch gekennzeichnet, dass** Erzeugen eines Zielbildes Folgendes einschließt:
Bestimmen optimierter Projektionsdaten der mindestens zwei Standardmaterialien durch Durchführen von Optimierungskorrektur an den Projektionsdaten der mindestens zwei Standardmaterialien, einschließend:
Erhalten eines Optimierungsparameters, der basierend auf Systemkalibrierungsdaten bestimmt wird; und
Bestimmen der optimierten Projektionsdaten der mindestens zwei Standardmaterialien durch Durchführen, basierend auf dem Optimierungsparameter, der Optimierungskorrektur an den Projektionsdaten der mindestens zwei Standardmaterialien; und
Erzeugen des Zielbildes basierend auf den optimierten Projektionsdaten der mindestens zwei Standardmaterialien.

11. System nach Anspruch 10, wobei das Bestimmen optimierter Projektionsdaten der mindestens zwei Standardmaterialien durch Durchführen von Optimierungskorrektur an den Projektionsdaten der mindestens zwei Standardmaterialien Folgendes einschließt:
Erhalten eines Optimierungsparameters, der basierend auf Systemkalibrierungsdaten bestimmt wird; und
Bestimmen der optimierten Projektionsdaten der mindestens zwei Standardmaterialien durch Durchführen, basierend auf dem Optimierungsparameter, von Optimierungskorrektur an den Projektionsdaten der mindestens zwei Standardmaterialien.

12. System nach Anspruch 10 oder Anspruch 11, wobei die Luftkorrekturtabelle erhalten wird durch:
Erhalten von aktuellen Luftdaten, die den Scandaten entsprechen;
Bestimmen von Referenzluftdaten, die der Systemkalibrierung entsprechen; und
Bestimmen der Luftkorrekturtabelle basierend auf den aktuellen Luftdaten und den Referenzluftdaten.

13. Nichtflüchtiges computerlesbares Medium, das mindestens einen Satz von Anweisungen umfasst, wobei, der mindestens eine Satz von Anweisungen, wenn er durch einen oder mehrere Prozessoren ausgeführt wird, bewirkt, dass die Rechenvorrichtung das CT-Bildgebungsverfahren nach einem der Ansprüche 1-9 durchführt.

## Revendications

1. Procédé d'imagerie par tomodensitométrie (TDM), comprenant :
l'obtention de données de numérisation d'un sujet cible, les données de numérisation étant acquises en scannant le sujet cible à l'aide d'un dispositif TDM ;
la détermination de données après correction atmosphérique en effectuant une correction atmosphérique sur les données de numérisation à l'aide d'une table de correction atmosphérique ;
la détermination des données de projection d'au moins deux matériaux standard en effectuant une décomposition du matériau de base sur les données après correction atmosphérique ;
la génération d'une image cible à partir des données de projection des au moins deux matériaux standard,
**caractérisé en ce que** la génération d'une image cible inclut :
la détermination de données de projection optimisées des au moins deux matériaux standard en effectuant une correction d'optimisation sur les données de projection des au moins deux matériaux standard, notamment :
l'obtention d'un paramètre d'optimisation déterminé à partir de données d'étalonnage du système ; et
la détermination des données de projection optimisées des au moins deux matériaux standard en effectuant, sur la base du paramètre d'optimisation, la correction d'optimisation sur les données de projection des au moins deux matériaux standard ; et
la génération de l'image cible à partir des données de projection optimisées des au moins deux matériaux standard.

2. Procédé selon la revendication 1, dans lequel le paramètre d'optimisation est déterminé par :
l'obtention des données d'étalonnage du système en numérisant au moins deux matériaux d'étalonnage du système, les données d'étalonnage du système incluant un taux de comptage de photons détecté par un détecteur du dispositif TDM et une longueur intégrale de trajet réelle des au moins deux matériaux d'étalonnage du système correspondant au taux de comptage de photons ;
la détermination d'une longueur intégrale de trajet estimée en intégrant le taux de comptage de photons des données d'étalonnage du système dans un modèle de réponse configuré pour la décomposition du matériau de base ; et
la détermination du paramètre d'optimisation en fonction de la longueur intégrale de trajet estimée et de la longueur intégrale de trajet réelle.

3. Procédé selon la revendication 2, dans lequel la détermination du paramètre d'optimisation basée sur la longueur intégrale de trajet estimée et la longueur intégrale de trajet réelle inclut :
la détermination d'une relation de correspondance entre les données de projection optimisées des au moins deux matériaux standard et les données de projection des au moins deux matériaux standard, en se basant sur la longueur intégrale de trajet estimée et la longueur intégrale de trajet réelle ; et
la détermination de la relation de correspondance comme paramètre d'optimisation.

4. Procédé selon la revendication 2 ou 3, dans lequel la détermination du paramètre d'optimisation basée sur la longueur intégrale de trajet estimée et la longueur intégrale de trajet réelle inclut :
la génération d'un modèle d'optimisation en effectuant, sur la base de la longueur intégrale de trajet estimée et de la longueur intégrale de trajet réelle, un processus d'apprentissage du modèle, la longueur intégrale de trajet réelle étant utilisée comme marqueur de la longueur intégrale de trajet estimée.

5. Procédé selon l'une quelconque des revendications 1-4, comprenant en outre :
la détermination si l'image cible satisfait à une première condition prédéfinie ;
en réponse à la constatation que l'image cible ne satisfait pas à la première condition prédéfinie, la mise à jour de la table de correction atmosphérique ;
la détermination des premières données après correction atmosphérique en effectuant, sur la base d'une table de correction atmosphérique mise à jour, une correction atmosphérique sur les données de numérisation ; et
la génération d'une première image cible basée sur les premières données après correction atmosphérique.

6. Procédé selon l'une quelconque des revendications 1-5, dans lequel la mise à jour du tableau de correction atmosphérique inclut :
l'obtention de données atmosphériques actuelles correspondant aux données de numérisation ;
l'obtention de données atmosphériques de référence correspondant à l'étalonnage du système ; et
la détermination du tableau de correction atmosphérique mis à jour en fonction des données atmosphériques actuelles et des données atmosphériques de référence.

7. Procédé selon la revendication 5 ou la revendication 6, comprenant en outre :
la détermination si la première image cible satisfait à la première condition prédéfinie ;
en réponse à la constatation que la première image cible ne satisfait pas à la première condition prédéfinie, la détermination d'un premier résultat d'étalonnage du système en réeffectuant l'étalonnage du système ; et
la génération d'une deuxième image cible basée sur le résultat du premier étalonnage du système.

8. Procédé selon l'une quelconque des revendications 1-6, dans lequel la génération d'une image cible basée sur les données de projection optimisées des au moins deux matériaux standard inclut :
la détermination d'un premier résultat de prédiction de la qualité d'image en saisissant les données de projection optimisées des au moins deux matériaux standard dans un modèle de prédiction de la qualité d'image ;
la détermination si le premier résultat de prédiction de la qualité d'image satisfait à une deuxième condition prédéfinie ;
en réponse à la constatation que le résultat de la prédiction de la qualité d'image ne satisfait pas à la deuxième condition prédéfinie, la mise à jour de la table de correction atmosphérique ;
la détermination des premières données après correction atmosphérique en effectuant, sur la base d'une table de correction atmosphérique mise à jour, une correction atmosphérique sur les données de numérisation ; et
la génération de l'image cible à partir des premières données après correction atmosphérique.

9. Procédé selon la revendication 8, dans lequel la génération de l'image cible à partir des premières données après correction atmosphérique inclut :
la détermination de premières données de projection d'au moins deux matériaux standard en effectuant une décomposition du matériau de base sur les premières données après correction atmosphérique ;
la détermination de premières données de projection optimisées des au moins deux matériaux standard en effectuant une correction d'optimisation sur les premières données de projection des au moins deux matériaux standard ;
la détermination d'un deuxième résultat de prédiction de la qualité d'image en saisissant les premières données de projection optimisées des au moins deux matériaux standard dans le modèle de prédiction de la qualité d'image ;
la détermination si le deuxième résultat de prédiction de la qualité d'image satisfait la deuxième condition prédéfinie ;
en réponse à la constatation que le deuxième résultat de prédiction de la qualité d'image ne satisfait pas à la deuxième condition prédéfinie, la détermination d'un premier résultat d'étalonnage du système en réeffectuant l'étalonnage du système ; et
la génération de l'image cible basée sur le premier résultat d'étalonnage du système.

10. Système d'imagerie par tomodensitométrie (TDM), comprenant :
un module d'acquisition de données de numérisation (910) configuré pour obtenir des données de numérisation d'un sujet cible, les données de numérisation étant acquises en numérisant le sujet cible à l'aide d'un dispositif TDM ;
un module de correction (920) configuré pour déterminer les données après correction atmosphérique en effectuant une correction atmosphérique sur les données de numérisation à l'aide d'une table de correction atmosphérique ;
un module de décomposition (930) configuré pour déterminer les données de projection d'au moins deux matériaux standard en effectuant une décomposition du matériau de base sur les données après correction atmosphérique ;
un module d'optimisation (940) configuré pour déterminer les données de projection optimisées des au moins deux matériaux standard en effectuant une correction d'optimisation sur les données de projection des au moins deux matériaux standard ; et
un module d'imagerie (950) configuré pour générer une image cible basée sur les données de projection optimisées des au moins deux matériaux standard,
**caractérisé en ce que** la génération d'une image cible inclut :
la détermination de données de projection optimisées des au moins deux matériaux standard en effectuant une correction d'optimisation sur les données de projection des au moins deux matériaux standard, notamment :
l'obtention d'un paramètre d'optimisation déterminé à partir de données d'étalonnage du système ; et
la détermination des données de projection optimisées des au moins deux matériaux standard en effectuant, sur la base du paramètre d'optimisation, la correction d'optimisation sur les données de projection des au moins deux matériaux standard ; et
la génération de l'image cible à partir des données de projection optimisées des au moins deux matériaux standard.

11. Système selon la revendication 10, dans lequel la détermination de données de projection optimisées des au moins deux matériaux de référence par correction d'optimisation des données de projection d'au moins deux matériaux de référence inclut :
l'obtention d'un paramètre d'optimisation déterminé à partir de données d'étalonnage du système ; et
la détermination des données de projection optimisées des au moins deux matériaux standard en effectuant, sur la base du paramètre d'optimisation, la correction d'optimisation sur les données de projection des au moins deux matériaux standard.

12. Système selon la revendication 10 ou la revendication 11, dans lequel le tableau de correction atmosphérique est obtenu par :
l'obtention de données atmosphériques actuelles correspondant aux données de numérisation ;
l'obtention de données atmosphériques de référence correspondant à l'étalonnage du système ; et
la détermination du tableau de correction atmosphérique en fonction des données atmosphériques actuelles et des données atmosphériques de référence.

13. Support non transitoire lisible par ordinateur, comprenant au moins un ensemble d'instructions, dans lequel lorsqu'il est exécuté par un ou plusieurs processeurs d'un dispositif informatique, le au moins un ensemble d'instructions amène le dispositif informatique à mettre en œuvre le procédé d'imagerie TDM selon l'une quelconque des revendications 1-9.
